# EUROPEAN PATENT APPLICATION

(11) **EP 4 632 755 A1**
(43) Date of publication of application: **15.10.2025**
(21) Application number: 25169001.2
(22) Date of filing: 08.04.2025
(51) Int. Cl.: G16H 20/40, G16H 40/63, G16H 50/50

(54) **MAPPING A CAVITY**

(30) Priority: 09.04.2024 US 202418630079
(71) Applicant: Biosense Webster (Israel) Ltd., 2066717 Yokneam (IL)
(72) Inventor: MASSARWI, Fady, 2066717 Yokneam (IL); KATZ, Natan Sharon, 2066717 Yokneam (IL); PELKA, Piotr Szymon, 2066717 Yokneam (IL)
(74) Representative: Carpmaels & Ransford LLP

(57) **Abstract**

A method, consisting of acquiring data representative of a volume of a cavity of an organ, presenting on a display an electroanatomical (EA) map of a surface, generated in response to the data, enclosing the volume, and receiving input from a user, the input including a selected section of the EA map. The method includes, in response to the user input, locking a portion of the volume to subsequent updates of the data when updating the EA map, the portion of the volume consisting of the selected section of the EA map.

## Description

### FIELD OF THE DISCLOSURE

This disclosure relates generally to mapping, and specifically to mapping a cavity.

### BACKGROUND

During a medical procedure on a cavity, such as a chamber of a heart, the chamber may be mapped initially, and then may be remapped to observe changes in the chamber due to the procedure. It is important that both initial and subsequent mappings are as efficient as possible.

### BRIEF DESCRIPTION OF THE DRAWINGS

The present disclosure will be understood from the following detailed description, taken in conjunction with the drawings in which:
Fig. 1 shows a catheter-based electrophysiology mapping and ablation system, according to an example of the present disclosure;
Fig. 2 is a flowchart of steps implemented in mapping the surface of a cavity of an organ of a patient, according to an example of the present disclosure; and
Figs 3A - 3I are diagrams illustrating the steps of the flowchart, according to an example of the present disclosure.

### DESCRIPTION OF EXAMPLES

### OVERVIEW

In a procedure on a chamber of the heart, such as an ablation procedure, the chamber is typically initially mapped so as to generate an electroanatomical (EA) map of the chamber. The mapping may be implemented using a Fast Anatomical Mapping (FAM) method, wherein a processor registers and acquires locations of voxels on the surface of the chamber visited by a catheter, and from the locations generates the EA map. During or after the voxel acquisition, errors in the surface may be corrected by "shaving" the surface, to remove incorrectly acquired voxels. The shaving may be performed manually, by a physician or a technologist assisting the physician, and/or automatically.

The chamber may be remapped, typically after the procedure has been performed. During the remapping, the shaved voxels may be reacquired, so that the shaving needs to be repeated. Repeating the shaving requires time, and may involve many map changes, as well as intermediate results. Both the time required and the map changes reduce the efficiency of the procedure.

Examples of the present disclosure improve the efficiency of the remapping. Voxels that were initially shaved, as well as voxels in proximity to the shaved voxels, are marked, and are locked so that during the remapping the catheter does not acquire the marked voxels. The processor then implements the remapping absent the locked voxels, so that shaving does not need to be repeated.

### SYSTEM DESCRIPTION

In the following description, like elements are identified by the same numeral, and are differentiated, where required, by having a letter attached as a suffix to the numeral.

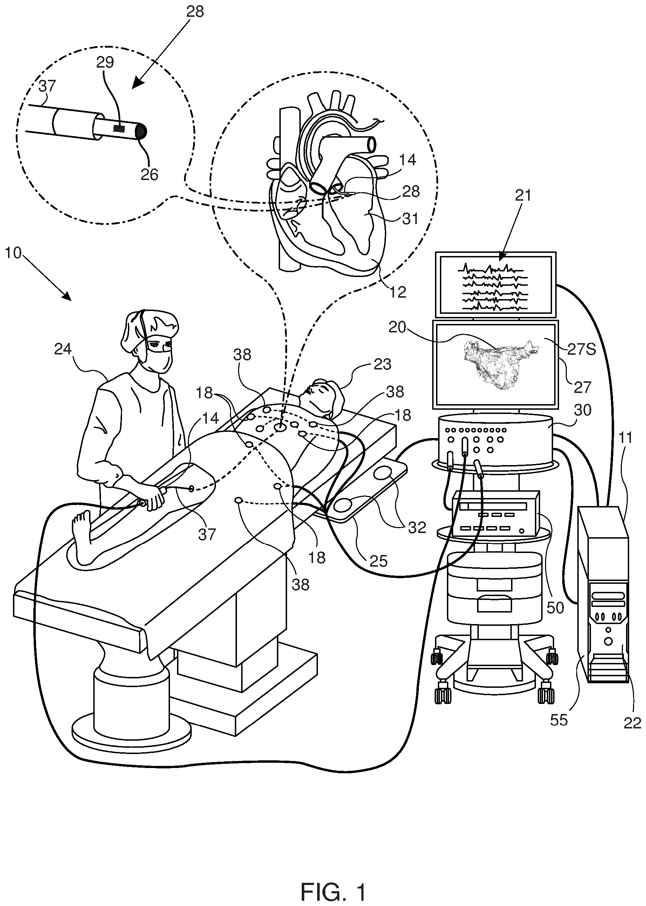

Reference is now made to Fig. 1 which shows a catheter-based electrophysiology mapping and ablation system 10, according to an example of the present disclosure. System 10 includes multiple catheters, which are percutaneously inserted by a physician 24 through the patient's vascular system into a chamber, such as a chamber 31, or the vascular structure of a heart 12. Typically, a delivery sheath catheter 37 is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters for mapping, catheters dedicated for ablating and/or catheters dedicated for both sensing and ablating. An example catheter 14 that is configured for sensing IEGM signals and for mapping is illustrated herein.

Catheter 14 is an exemplary focal catheter that includes at least one electrode 26 distributed on a distal tip 28 of the catheter. Catheter 14 additionally includes at least one position sensor 29 embedded in or near distal tip 28 for tracking position and orientation of the distal tip. In a disclosed example, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. The real time position of distal tip 28 of catheter 14 may be tracked based on magnetic fields generated with location pad 25 and sensed by magnetic based position sensor 29. The real time position comprises the location of the position sensor and the orientation of the position sensor. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on a patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) that may be captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, a processor 22 with memory or storage with appropriate operating software loaded therein, and user interface capability. Workstation 55 may provide multiple functions, optionally including (1) modeling the endocardial anatomy in three-dimensions (3D) and rendering a model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of distal tip 28 within the heart chamber, and (4) displaying on display device 27 sites of interest such as places where ablation energy has been applied. Display device 27 has a screen 27S. One commercial product embodying elements of the system 10 is available as the CARTO^{™} 3 System, available from Biosense Webster, Inc., 31A Technology Drive, Irvine, CA 92618.

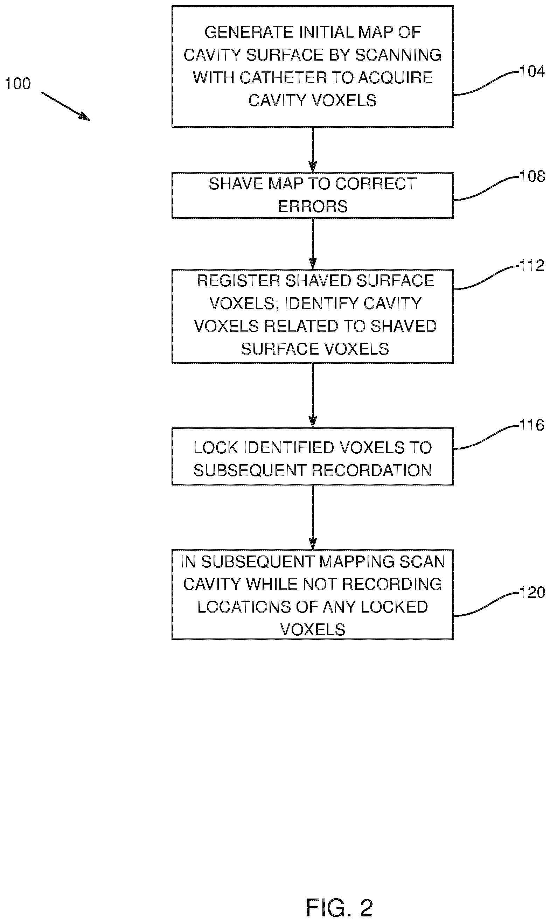

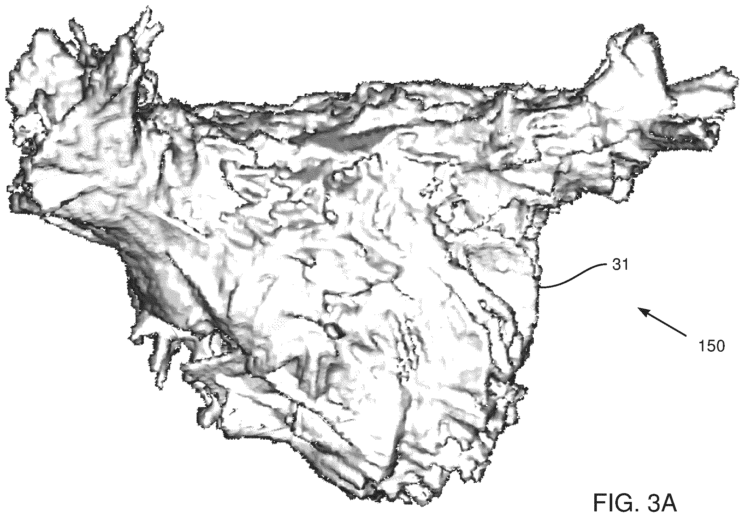

Fig. 2 is a flowchart 100 of steps implemented in mapping the surface of a cavity 31 of an organ 12 of patient 23, and Figs 3A - 3I are diagrams illustrating the steps, according to an example of the present disclosure. In the following description organ 12 is assumed to comprise heart 12, and may also be referred to herein as heart 12, and cavity 31 is assumed to comprise chamber 31, and may also be referred to herein as chamber 31, and those having ordinary skill in the art will be able to adjust the description, *mutatis mutandis,* for other cavities and/or organs of patient 23.

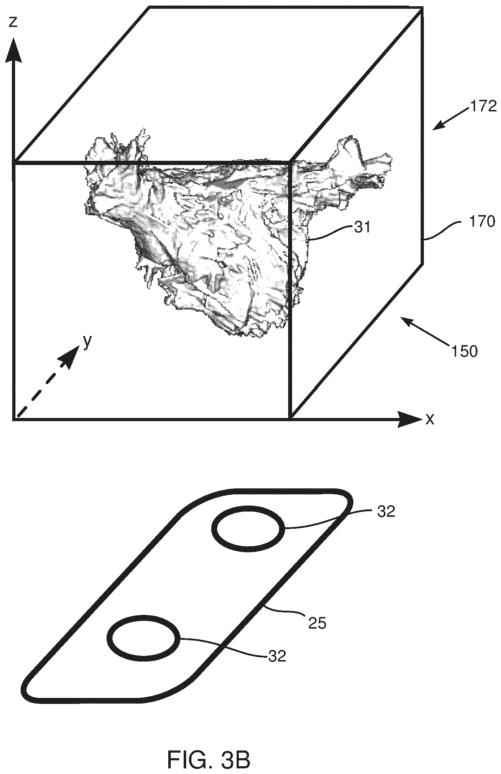

Fig. 3A schematically illustrates an initial map 150 of the surface of chamber 31, and map 150 is also herein termed surface 150. In producing the mapping, and as illustrated in Fig. 3B, chamber 31 is assumed to be in a cuboid volume 170 comprised of rectangular parallelepiped voxels, the cuboid volume being in a frame of reference defined by location pad 25. Cuboid volume 170 has been drawn on Cartesian xyz axes. In one example the voxels of the cuboid volume 170 are xyz cubes having an edge of 0.8 mm, but in other examples the voxels may have a different edge value, and/or may not be cubes. Chamber 31 is assumed to be comprised of cavity xyz voxels 172, the cavity voxels being a subset of the voxels of cuboid volume 170.

To produce initial map 150, in a data acquisition step 104, physician 24 inserts distal tip 28 into chamber 31, and moves the distal tip within the chamber. While the distal tip is in the chamber, processor 22 records the multiple locations of position sensor 29, as well as the orientation of the sensor at each location. The dimensions of distal tip 28 are known, and are provided to processor 22. From the distal tip dimensions, and from the recorded locations and orientations of sensor 29, processor 22 identifies and acquires a set of voxels of volume 170, herein also termed data voxels, corresponding to the volumes occupied by the distal tip. It will be understood that the acquired set of data voxels is a subset of cavity voxels 172.

Once processor 22 has acquired the set of voxels corresponding to the multiple positions of the distal tip, the processor analyzes the voxels to find a three-dimensional (3D) net, of line segments connecting voxels, that encloses the acquired voxels. The net comprises a set of line segments joining outer data voxels, herein termed surface voxels, of the acquired voxels. In one example the surface voxels and the set of line segments joining the surface voxels may be found using a ball-pivot algorithm, which forms the surface voxels and the line segments into a set of connected triangles. In a disclosed example physician 24 may be able to set the resolution of the net produced, essentially changing the size of the triangles of the net, by changing the diameter of the ball.

The 3D net produced encloses all acquired data voxels, except for the surface voxels which are joined by the net.

Processor 22 uses interpolation to fill the triangles of the net so as to calculate a 3D surface, and presents the calculated surface to physician 24 on device 27. The presented surface corresponds to the calculated surface of chamber 31.

In a "shaving" step 108, physician 24 uses tools of device 27 to edit the surface generated in step 104. Typically the generated surface has errors which are apparent to the physician, the errors being caused, for example, by the beating of heart 12, the respiration of patient 23, and/or tenting of the wall of chamber 31 when the distal tip contacts the wall. The tools used to modify the surface enable physician 24 to identify to processor 22 regions of the surface which appear to be incorrect. For each incorrect surface region identified, processor 22 registers the 3D locations of the surface voxels associated with the region, recalculates the net assuming the registered surface voxels are absent, and presents a recalculated 3D surface based on the recalculated net to physician 24 on device 27.

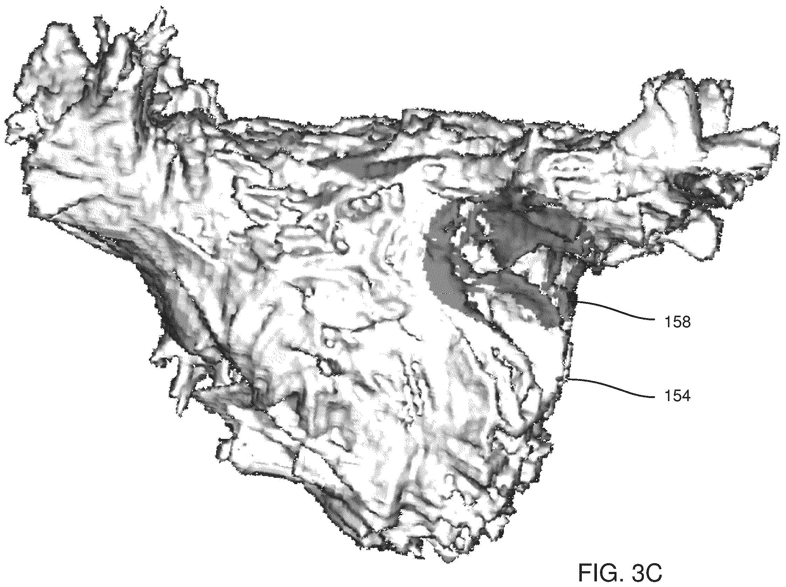

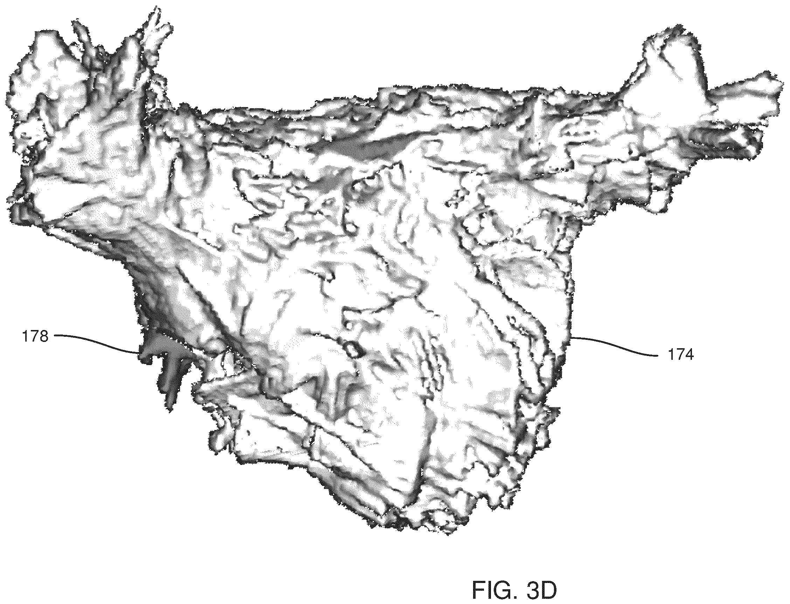

Fig. 3C schematically illustrates a first recalculated surface 154; physician 24 has modified initial surface 150 in a region 158, and processor 22 recalculates the surface, as described above. Fig. 3D schematically illustrates a second recalculated surface 174; physician 24 has modified initial surface 150 in a region 178, and processor 22 recalculates the surface, as described above.

Examples of the present disclosure assume that at some time after the implementation of steps 104 and 108, the initial mapping of chamber 31, performed in step 104, is repeated in a subsequent mapping operation.

Prior to the subsequent mapping, in an identification step 112, processor 22 records the 3D locations of the surface voxels associated with the modified initial surface, i.e., surface voxels associated with region 158, and surface voxels associated with region 178, and identifies a set of cavity voxels proximate to the recorded surface voxels. As is described below, the identified cavity voxels, proximate to the recorded surface voxels, are "locked" so that processor 22 does not record locations of the locked voxels during the subsequent mapping.

Examples of the present disclosure provide two alternative methods for identifying voxels proximate to the recorded surface voxels: a morphological closing method and an extrusion method. Both methods are described below: the morphological closing method is described with reference to Fig. 3C, the extrusion method is described with reference to Fig. 3D.

### Morphological Closing Method

In the morphological closing method, processor 22 applies a dilation and erosion algorithm to each of the recorded surface voxels. The algorithm enables the processor to close gaps between surface voxels that are separated from each other by a predetermined distance. After applying the algorithm and identifying gap voxels forming the gaps, processor 22 then checks for closed sub-volumes, i.e., voids, within a volume comprising the set of surface voxels and the identified gap voxels. The processor identifies the voxels in each void as filling voxels.

In a disclosed example, the processor uses as a kernel for the dilation and erosion algorithm a cube having five voxels as its edge length, so that the kernel comprises a cube of 125 voxels. For the dilation part of the algorithm, the processor dilates every recorded surface voxel, producing for each given surface voxel a set of 124 dilation voxels surrounding the given voxel.

For the erosion part of the algorithm, the kernel is used to check if the dilation voxels produced by the dilation are retained. To check for retention, the processor applies the kernel to every voxel produced by the dilation, and rejects a given one of these dilation voxels unless the kernel is completely filled by other dilation voxels or surface voxels.

At completion of the algorithm, there is a resultant set of voxels, some, but not necessarily all, of which may close gaps between surface voxels. The voxels in the resultant set are also herein termed gap voxels.

As a first example of the application of the algorithm, in the case of a single surface voxel, only the single surface voxel comprises the resultant set after implementation of the algorithm. As a second example of the algorithm, for two surface voxels separated by a single voxel, the two surface voxels and the separating voxel comprise the resultant set after implementation of the algorithm.

After applying the dilation and erosion algorithm to all the recorded surface voxels, the processor analyzes the resultant set of voxels, i.e., the gap voxels, for closed three-dimensional voids in the set. In a disclosed embodiment the analysis may be performed by applying a flood fill algorithm to the combined set of surface and gap voxels in cuboid volume 170, and recording which voxels, other than the voxels of the combined set, are not filled. It will be understood that the voxels that are not filled correspond to voxels of voids in the combined set of voxels, and these voxels are herein termed void voxels.

To complete identification step 112 for the morphological closing method, processor 22 saves the locations of the surface voxels recorded in step 108, and of the gap voxels and the void voxels found in step 112.

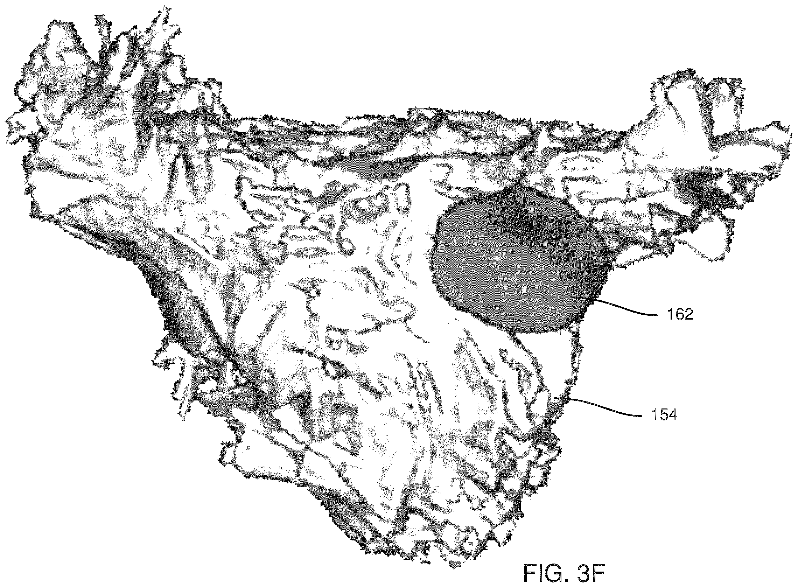

In a disclosed example a region corresponding to the locations of the saved voxels may be displayed on recalculated surface 154 to physician 24, using device 27. Fig. 3F schematically illustrates a region 162 corresponding to the saved voxels derived from region 158.

### Two-Dimensional Illustration of Morphological Closing Method

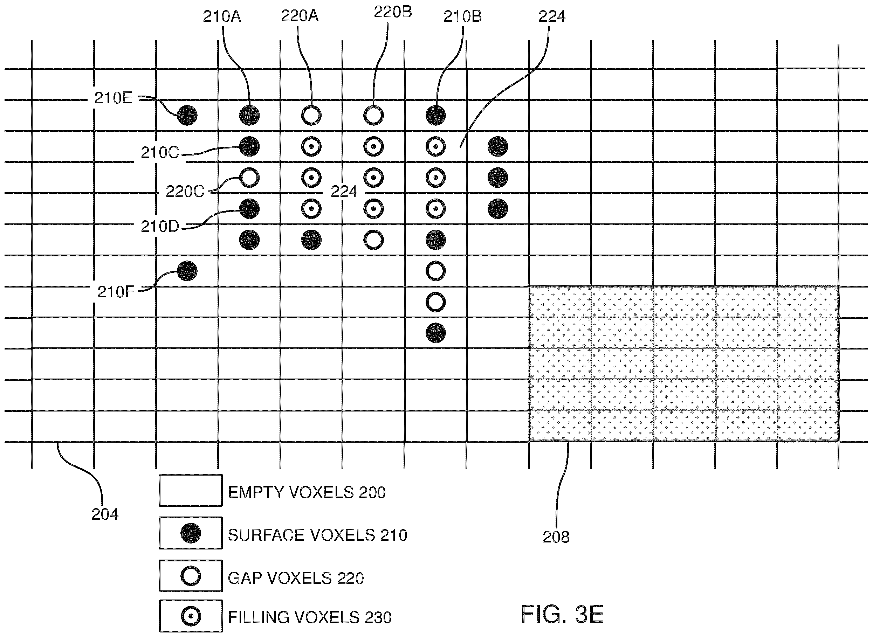

As stated above, in identification step 112 processor 22 analyzes the three-dimensional surface voxels recorded in step 108. Fig. 3E provides a two-dimensional illustration of the stages of the analysis of step 112. The illustration assumes that processor 22 has acquired and recorded in step 108 surface voxels 210. Surface voxels 210 are assumed to be located in a section of a two-dimensional (2D) rectilinear grid 204 that is part of cuboid volume 170, and the rectangles of the grid show possible other voxels 200, also termed empty voxels 200 of the grid.

In the 2D example of Fig. 3E, the processor is assumed to use a square kernel 208 of 5×5 voxels when applying the dilation and erosion algorithm. The gap voxels 220 that result from applying the kernel are shown in the figure. As is illustrated in the figure, the algorithm generates gap voxels 220A and 220B between surface voxels 210A and 210B, and also generates a gap voxel 220C between surface voxels 210C and 210D. (Note that there are no gap voxels generated between surface voxels 210E and 210F.)

Once processor 22 has scanned all surface voxels 210 acquired in step 108, and the processor applies a fill flood algorithm to grid 204. The algorithm detects a void 224 in the grid, and the processor records the locations of filling voxels 230 in the void.

### Extrusion Method

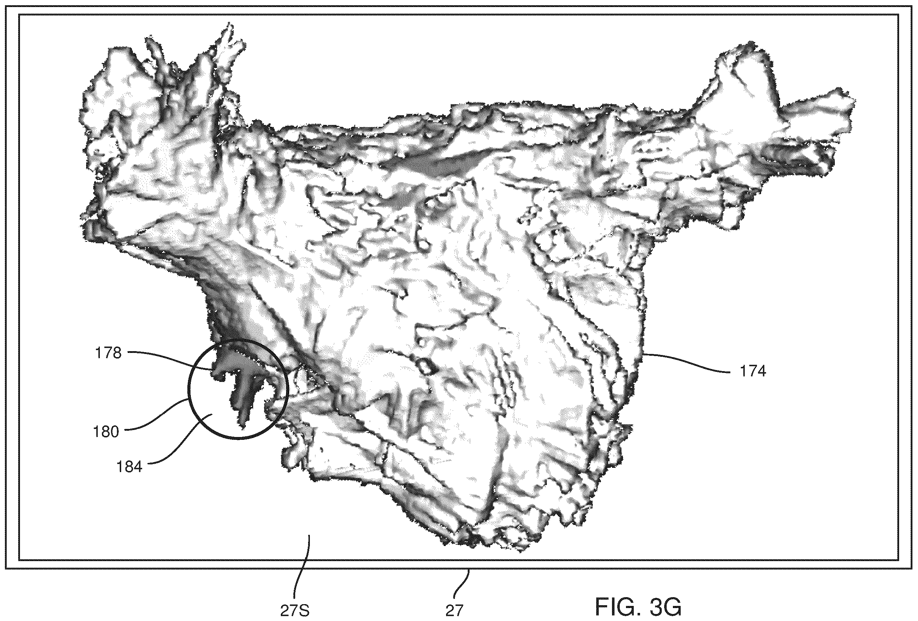

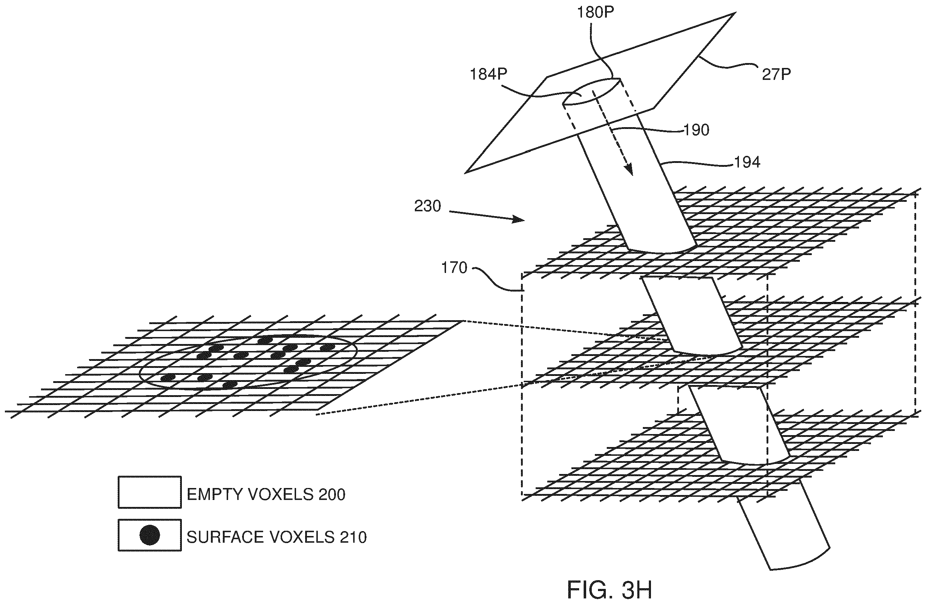

Figs. 3G and 3H schematically illustrate the extrusion method. Fig. 3G shows surface 174 as it is presented on screen 27S of device 27, and Fig. 3H illustrates the process applied by processor 22 in implementing the method. In contrast to the morphology method described above, in the extrusion method a user, herein assumed to be physician 24, delineates on surface 174, using tools of device 27, a bound 180 on surface 174 that typically surrounds region 178. Bound 180 forms the perimeter surrounding an enclosed region 184. The enclosed region may be in any convenient shape selected by physician 24, and in a disclosed example, illustrated in Fig. 3G, bound 180 and enclosed region 184 are circular.

Fig. 3H schematically illustrates a plane 27P, corresponding to the plane of screen 27S of device 27, Representations 180P and 184P, of bound 180 and enclosed region 184, have been drawn on plane 27P. Fig. 3H also illustrates planes of cuboid volume 170. A callout of one of the planes illustrates surface voxels 210 that are present in the plane. At least some other planes of the cuboid typically also have surface voxels 210. In all planes there are typically empty voxels 200.

As illustrated in Fig. 3H, processor 22 extrudes enclosed region 184 along a vector 190. Vector 190 is orthogonal to plane 27P, i.e., to the plane of screen 27S of device 27 and is in a direction into the screen. The extrusion of region 184 forms a prism 194, with the prism base corresponding to region 184, and it will be understood that when bound 180 is circular, prism 194 comprises a right cylinder.

Processor 22 registers all the voxels within prism 194, i.e., surface voxels 210 and empty voxels 200, and records them as filling voxels 230.

To complete identification step 112 for the extrusion method, processor 22 saves the locations of all filling voxels 230 in prism 194.

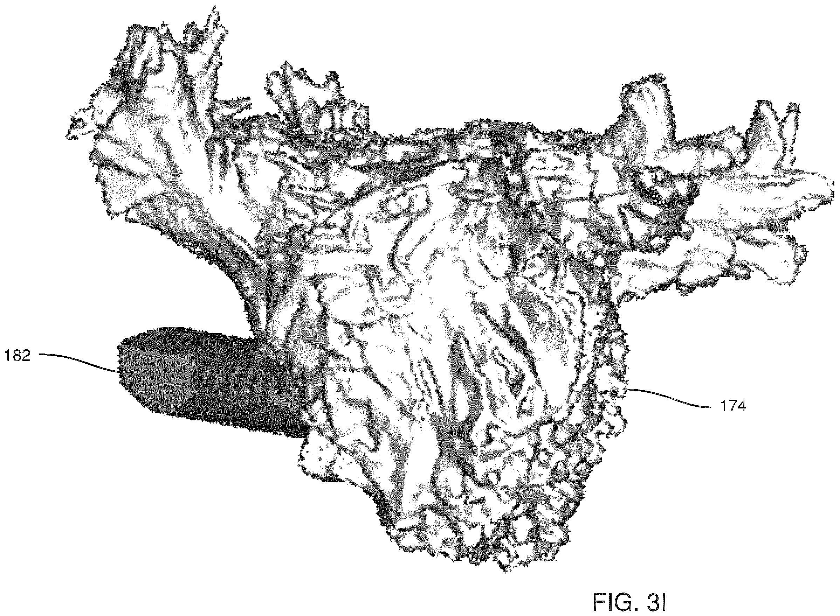

In a disclosed example a region corresponding to the locations of the saved voxels may be displayed on surface 174 to physician 24, using device 27. Fig. 3I schematically illustrates a region 182 corresponding to the saved voxels derived from region 178 (Fig. 3G).

Returning to flowchart 100, in a locking step 116, processor 22 assigns all voxels identified and saved in step 112 as locked voxels, i.e., as voxels that, in a subsequent scanning and mapping of chamber 31, are not considered in calculating the surface of the chamber.

In a final, subsequent mapping step 120 of the flowchart, physician 24 remaps chamber 31, using distal tip 28, substantially as described above for data acquisition step 104. During the remapping, the processor does not record the locations of any of the voxels that have been locked in step 116, even if an acquired voxel is identified as corresponding to the volume occupied by distal tip 28.

The remapping generates a new surface, which may be displayed to physician 24 on device 27. Because of the voxels that have been locked in step 116, the new surface does not require shaving.

### Examples

Example 1. A method, comprising:
acquiring data representative of a volume of a cavity (31) of an organ;
presenting, on a display (27), an electroanatomical (EA) map (20) of a surface, generated in response to the data, enclosing the volume;
receiving input from a user, the input comprising a selected section of the EA map; and
in response to the user input, locking a portion of the volume to subsequent updates of the data when updating the EA map, the portion of the volume comprising the selected section of the EA map.

Example 2. The method according to example 1, wherein acquiring the data comprises acquiring locations of a set of cavity voxels comprising the volume, and analyzing the locations to determine outer voxels of the set, the outer voxels corresponding to the surface enclosing the volume, and wherein locking the portion of the volume comprises selecting a subset of the outer voxels in response to the user input and not recording locations of the subset when updating the EA map.

Example 3. The method according to example 2, wherein locking the portion of the volume further comprises selecting a predefined group of the cavity voxels in proximity to the subset.

Example 4. The method according to example 3, wherein selecting the predefined group comprises applying a dilation and erosion algorithm to the subset so as to generate a resultant set of the cavity voxels.

Example 5. The method according to example 4, wherein selecting the predefined group comprises analyzing the resultant set to determine one or more voids therein, and wherein the predefined group comprises the cavity voxels within the one or more voids.

Example 6. The method according to example 3, wherein receiving the input from the user comprises the user delineating a bound surrounding an enclosed two-dimensional (2D) region on a representation of the EA map of the surface, and wherein the predefined group comprises the cavity voxels within a prism having a base as the closed 2D region.

Example 7. The method according to example 1, wherein the organ comprises a heart.

Example 8. Apparatus, comprising:
a display (27); and
a processor (22), configured to acquire data representative of a volume of a cavity (31) of an organ,
present, on the display, an electroanatomical (EA) map (20) of a surface, generated in response to the data, enclosing the volume,
receive input from a user, the input comprising a selected section of the EA map, and
in response to the user input, lock a portion of the volume to subsequent updates of the data when updating the EA map, the portion of the volume comprising the selected section of the EA map.

The examples described above are cited by way of example, and the present disclosure is not limited by what has been particularly shown and described hereinabove. Rather the scope of the disclosure includes both combinations and sub-combinations of the various features described hereinabove, as well as variations and modifications thereof which would occur to persons skilled in the art upon reading the foregoing description and which are not disclosed in the prior art.

## Claims

1. A method, comprising:
acquiring data representative of a volume of a cavity (31) of an organ (12);
presenting, on a display (27), an electroanatomical (EA) map (20) of a surface (150), generated in response to the data, enclosing the volume;
receiving input from a user, the input comprising a selected section (158, 178) of the EA map; and
in response to the user input, locking a portion of the volume to subsequent updates of the data when updating the EA map, the portion of the volume comprising the selected section of the EA map.

2. The method according to claim 1, wherein acquiring the data comprises acquiring locations of a set of cavity voxels (172) comprising the volume, and analyzing the locations to determine outer voxels of the set, the outer voxels corresponding to the surface enclosing the volume, and wherein locking the portion of the volume comprises selecting a subset of the outer voxels in response to the user input and not recording locations of the subset when updating the EA map.

3. The method according to claim 2, wherein locking the portion of the volume further comprises selecting a predefined group of the cavity voxels in proximity to the subset.

4. The method according to claim 3, wherein selecting the predefined group comprises applying a dilation and erosion algorithm to the subset so as to generate a resultant set of the cavity voxels.

5. The method according to claim **4,** wherein selecting the predefined group comprises analyzing the resultant set to determine one or more voids (224) therein, and wherein the predefined group comprises the cavity voxels within the one or more voids.

6. The method according to claim 2 or claim 3, wherein receiving the input from the user comprises the user delineating a bound (180) surrounding an enclosed two-dimensional (2D) region (184) on a representation of the EA map of the surface, and wherein the predefined group comprises the cavity voxels within a prism (194) having a base as the closed 2D region.

7. The method according to any one of claims 1-6, wherein the organ comprises a heart (12).

8. Apparatus, comprising:
a display (27); and
a processor (22), configured to
acquire data representative of a volume of a cavity (31) of an organ (12),
present, on the display, an electroanatomical (EA) map (20) of a surface (150), generated in response to the data, enclosing the volume,
receive input from a user, the input comprising a selected section (158, 178) of the EA map, and
in response to the user input, lock a portion of the volume to subsequent updates of the data when updating the EA map, the portion of the volume comprising the selected section of the EA map.

9. The apparatus according to claim 8, wherein acquiring the data comprises acquiring locations of a set of cavity voxels (172) comprising the volume, and analyzing the locations to determine outer voxels of the set, the outer voxels corresponding to the surface enclosing the volume, and wherein locking the portion of the volume comprises selecting a subset of the outer voxels in response to the user input and not recording locations of the subset when updating the EA map.

10. The apparatus according to claim 9, wherein locking the portion of the volume further comprises selecting a predefined group of the cavity voxels in proximity to the subset.

11. The apparatus according to claim 10, wherein selecting the predefined group comprises applying a dilation and erosion algorithm to the subset so as to generate a resultant set of the cavity voxels.

12. The apparatus according to claim 11, wherein selecting the predefined group comprises analyzing the resultant set to determine one or more voids (224) therein, and wherein the predefined group comprises the cavity voxels within the one or more voids.

13. The apparatus according to any claim 9 or claim 10, wherein receiving the input from the user comprises the user delineating a bound (180) surrounding an enclosed two-dimensional (2D) region (184) on a representation of the EA map of the surface, and wherein the predefined group comprises the cavity voxels within a prism (194) having a base as the closed 2D region.

14. The apparatus according to any one of claims 8-13, wherein the organ comprises a heart (12).
